# EUROPEAN PATENT APPLICATION

(11) **EP 3 622 889 A1**
(43) Date of publication of application: **18.03.2020**
(21) Application number: 18306202.5
(22) Date of filing: 14.09.2018
(51) Int. Cl.: A61B 5/08, A61B 8/08

(54) **METHOD, DEVICE AND APPARATUS FOR MEASURING DIAPHRAGMATIC FUNCTIONAL PARAMETERS**

(71) Applicant: Association Institut de Myologie, 75013 Paris (FR); Institut National de la Santé et de la Recherche Médicale (INSERM), 75013 Paris 13 (FR); Sorbonne Université, 75006 Paris (FR); ASSISTANCE PUBLIQUE, HOPITAUX DE PARIS, 75004 Paris (FR)
(72) Inventor: BACHASSON, Damien, 75012 PARIS (FR); HOGREL, Jean-Yves, 92120 MONTROUGE (FR); DRES, Martin, 94240 L'HAY LES ROSES (FR); GENNISSON, Jean-Luc, 95000 CERGY (FR); SIMILOWSKI, Thomas, 92130 ISSY LES MOULINEAUX (FR)
(74) Representative: IPAZ

(57) **Abstract**

The invention relates to a method for measuring one or more diaphragmatic functional parameters, said method comprising the steps consisting of:
- a) a stimulation step by which a stimulation of the diaphragm is provided to generate a movement of one or more parts of the diaphragm,
- b) during the movement of said one or more parts of the diaphragm, imaging one or more parts of the diaphragm over time through an imaging step comprising the steps of emitting unfocused ultrasound waves, detecting ultrasound waves reflected and/or scattered by organic tissues, processing the reflected and/or scattered ultrasound waves over time to generate images by technical means,
- c) processing, by technical means, images previously acquired during the imaging step to measure one or more movements of the diaphragm over time, and/or a propagation of a movement through the diaphragm over time, and/or a propagation speed of a movement through the diaphragm over time, and/or one or more movements of different parts of the diaphragm over time, and/or an amplitude of a movement of the diaphragm over time, and/or a time separating the stimulation of the diaphragm from the occurrence of a movement of the diaphragm associated to said stimulation,
- d) based on one or more measurements of the processing step, determining one or more functional parameters of the diaphragm by technical means.

## Description

### Field of the invention

The present invention relates to a method, a device and an apparatus for measuring diaphragmatic functional parameters of a diaphragm of a human or an animal. The present invention is in the field of medicine, biology and physiology. The present invention is non-invasive, may be apply to all type of populations and may be used to asses, inter alia, diaphragm effort, diaphragm function, diaphragm work and diaphragm dysfunctions.

The invention can be used as part of feedback loop for ventilatory support, exercise monitoring programs, physiotherapy and rehabilitation, and animal model for research programs. The invention can also be used from the moment a diaphragmatic dysfunction is suspected. The invention can be implemented on patient under assisted, spontaneous or volitional ventilation or during apnea.

### Background to the invention

Diaphragm is the principal muscle of respiration. Diaphragmatic dysfunctions are often benign but may result from affections processes that interfere with diaphragmatic innervation, contractile properties, or mechanical coupling to the chest wall. Dysfunctions of the diaphragm can be associated with the presence of disease, dyspnea, reduced exercise capacity, sleep disturbances and, in the more severe cases, have a negative impact on survival.

Prior art discloses a technic regarded as the gold standard for the assessment of diaphragm function. This technic relies on the measurement of transdiaphragmatic pressure (Pdi). Pdi is defined as the difference between pleural and abdominal pressures that are inferred from pressures in the lower esophagus (Pes) and stomach (Pga), respectively. The measurement of Pes and Pga is most frequently achieved by passing a pair of balloon catheters through the nose, following local anesthesia of the nasal mucosa and pharynx. Although Pdi is specific of diaphragm activation, it is invasive and indirectly reflects the intensity of diaphragm contraction.

Another prior art technic is the surface electromyography of the diaphragm (EMGdi). This technic is noninvasive but may be contaminated by other respiratory muscles (and cardiac artifacts) and exhibits limited validity for assessing diaphragm activity.

Therefore, prior art does not comprise any available technic for the assessment of diaphragmatic functional parameters which is accurate and noninvasive. The need of such a technic is critical.

### Summary of the invention

An object of the invention is to provide a noninvasive technic to assess at least one functional parameter of a diaphragm of a human or an animal.

To this end, according to a first aspect of the invention, there is provided a method for measuring one or more diaphragmatic functional parameters of a diaphragm of a human or an animal. The method comprises the steps consisting of:
- a) a stimulation step by which a stimulation of the diaphragm is provided to generate a movement of one or more parts of the diaphragm,
- b) during the movement of said one or more parts of the diaphragm, imaging one or more parts of the diaphragm over time through an imaging step comprising the steps of:
   - emitting at least 100 unfocused ultrasound waves per second towards a region of the human or the animal comprising the one or more parts of the diaphragm to be imaged during the imaging step,
   - detecting ultrasound waves reflected and/or scattered by organic tissues of the human or the animal located in the region,
   - processing the reflected and/or scattered ultrasound waves over time to generate images by technical means,
- c) processing, by technical means, images previously acquired during the imaging step to measure:
   - one or more movements of a given part of the diaphragm over time, and/or
   - a propagation of a movement from said one or more parts of the diaphragm to one or more adjacent parts over time, and/or
   - a propagation speed of the movement from said one or more parts of the diaphragm to one or more adjacent parts over time, and/or
   - one or more movements of different parts of the diaphragm over time, and/or
   - an amplitude of a movement of one or different parts of the diaphragm over time, and/or
   - a time separating the stimulation of the diaphragm from the occurrence of a movement of the diaphragm associated to said stimulation,
- d) based on one or more measurements of the processing step, determining one or more functional parameters of the diaphragm by technical means.

The stimulation according to the invention may be defined as an artificial stimulation as opposed to a natural stimulation. The natural stimulation comprises spontaneous and volitional respiratory maneuvers. The stimulation according to the invention does not comprise assisted ventilation or life support.

The movement of one or more parts of the diaphragm may be a movement of the entire diaphragm.

The stimulation may be directly applied to the diaphragm. The stimulation directly applied to the diaphragm may generate a local motion of one or more parts the diaphragm. The local motion of one or more parts of the diaphragm generated by the stimulation may propagate through a part or through the entire diaphragm.

The artificial stimulation of the diaphragm may encompass muscular responses of the diaphragm.

The movement of one or more parts of the diaphragm generated by the stimulation may arise from the diaphragm contraction.

Functional parameters may be understood as parameters that can be monitor, notably over time, and that can be used to characterize diaphragm functioning.

The stimulation according to the invention may comprise one stimulus or several stimuli or periodic stimuli.

The functional parameters of the diaphragm may comprise a contractility and/or a diaphragmatic pressure and/or a diaphragm function and/or a diaphragmatic work and/or a nerve conduction velocity and/or a spatial organization of muscle fascicle(s).

In this summary of the invention, the expression technical means may comprise, preferably only, at least one computer, a central and/or a calculation unit and/or a processing unit, an analog electronic circuit (preferably dedicated), a digital electronic circuit (preferably dedicated), and/or a microprocessor (preferably dedicated), and/or software means.

In this summary of the invention, the term "determining" may also be defined as assessing or estimating a parameter or a value.

According to a first variant of the method, the stimulation of the diaphragm may be an electrical or a magnetic stimulation.

The electrical and/or magnetic stimulation may be performed on the nervous system through:
- transcranial stimulation (supraspinal levels), and/or
- cervicomedullary (spinal) stimulation, and/or
- phrenic roots stimulation, and/or
- one or two of the main trunks of the phrenic nerve, and/or
- stimulation the diaphragm itself.

The electrical and/or magnetic stimulation may be applied during ventilation of the human or the animal. Namely, the human or the animal may be under spontaneous or volitional or respiratory maneuvers or under assisted ventilation or life support when the electrical and/or magnetic stimulation are applied to said human or animal.

According to the first variant, the processing step c) may further comprise a measure of the amplitude of the movement of the diaphragm. Preferably, the determination of the amplitude of the movement of the diaphragm may be based on an intensity of the stimulation.

The linear relation between the intensity of the stimulation and the amplitude of the movement of the diaphragm, generated by the stimulation, may be used to determine the amplitude of the movement based on the sole value of the intensity of the stimulation applied.

According to the first variant, the determining step d) further may comprise a determination of a nerve conduction velocity.

The nerve conduction velocity may be observed based on the time separating the stimulation of the diaphragm from the occurrence of a movement of the diaphragm associated to said stimulation. A determination of a nerve conduction disorder is merely the observation of an anomaly of nerve conduction based on a nerve conduction delay.

According to the first variant, the determining step d) may comprise a determination of:
- a contractility of the diaphragm, and/or
- a localized paralysis of the diaphragm.

Preferably, the determination of the contractility of the diaphragm, and/or a localized paralysis of the diaphragm, may be based on the propagation speed of a movement through the diaphragm.

The determination of the contractility of the diaphragm, and/or a localized paralysis of the diaphragm, may be based on:
- the propagation speed of a movement through the diaphragm, and/or
- the amplitude of the movement of the diaphragm, and/or
- a stiffness of the diaphragm.

The linear relation between the stiffness of the diaphragm and the propagation speed of a movement through the diaphragm, generated by the stimulation, may be used to determine the contractility.

The stiffness of one or more parts of the diaphragm, or of the entire diaphragm, may be determined based on the propagation speed of a movement through the diaphragm. The stiffness of one or more parts of the diaphragm, or of the entire diaphragm, may be determined based on the amplitude of the movement of the diaphragm.

According to the first variant, during the processing step c), images of diagram tissue previously acquired during the imaging step b), may be processed, by technical means, to measure the movement of said diaphragm tissue. A velocity profile of the diaphragm tissue is equivalent to a propagation of a movement from one or more parts of the diaphragm to one or more adjacent parts over time.

During the processing step c), the velocity profile of the diaphragm tissue may be processed, by technical means, to measure:
- peak heights being indicative of a contractility of the diaphragm, and/or
- peak areas being indicative of a contractility of the diaphragm, and/or
- peak widths being indicative of a contractility of the diaphragm, and/or
- peak slopes being indicative of a contractility of the diaphragm, and/or
- time to peaks being indicative of a contractility of the diaphragm, and/or
- an halftime relaxation being indicative of a contractility of the diaphragm, and/or
- an integral of diaphragm displacement within the inspiratory time over time being indicative of a diaphragmatic work, and/or
- a diaphragm displacement-time index computed as the product of a mean diaphragm displacement per breath within the inspiratory time and the ratio between an inspiratory time and the total respiratory cycle time being indicative of diaphragm function.

According to a second variant:
- the stimulation step a) may further comprise a mechanical stimulation and/or an acoustic stimulation of one or more parts of the diaphragm generating a mechanical wave and/or an acoustic wave at said given part, the mechanical wave and/or the acoustic wave propagating towards adjacent parts of said given part,
- the imaging step b) may further comprise imaging said given part and said adjacent parts through which the mechanical wave and/or the acoustic wave propagate(s).

According to the second variant, the mechanical wave and/or the acoustic stimulation may be an ultrasonic stimulation, said ultrasonic stimulation comprising an emission of one or more focused ultrasound waves per second towards a given part of the diaphragm, said one or more focused ultrasound waves generating an elastic shear wave at said given part, the elastic shear wave propagating towards adjacent parts of said given part.

According to the second variant, the processing step c) may further comprise a measure of a propagation velocity of the shear wave.

The propagation velocity of the shear wave is equivalent to a propagation of a movement from one or more parts of the diaphragm to one or more adjacent parts over time.

According to the second variant, the determining step d) may further comprise a determination of a contractility of the diaphragm. Preferably, the determination of the contractility of the diaphragm may be based on the propagation velocity of the shear wave.

A contractility of one or more parts of the diaphragm, or of the entire diaphragm, may be determined based on the propagation velocity of the shear wave.

The contractility of the diaphragm may be determined based on a stiffness of the diaphragm.

The linear relation between the stiffness of the diaphragm and the propagation velocity of the shear wave, generated by the stimulation, may be used to determine the contractility.

The stiffness of one or more parts of the diaphragm, or of the entire diaphragm, may be determined based on the propagation velocity of the shear wave.

According to the second variant, the stimulation step a) may be performed during ventilation of the human or the animal.

Namely, the human or the animal may be under spontaneous or volitional or respiratory maneuvers or under assisted ventilation or life support when the mechanical and/or acoustic stimulations is applied to said human or animal.

According to the second variant, the determining step d) may further comprise a determination of a diaphragm work.

According to the second variant, the determining step d) may further comprise a determination of a diaphragm activity. Preferably, the determining step d) is performed during ventilation of the human or the animal. Preferably, the determination of the diaphragm activity is based on the propagation velocity of the shear wave. Preferably, the determination of the diaphragm activity is based on the stiffness of the diaphragm. The linear relation between the stiffness of the diaphragm and the propagation velocity of the shear wave, generated by the stimulation, may be used to determine the diaphragm activity.

According to the second variant, the determining step d) may further comprise a determination of a transdiaphragmatic pressure. Preferably, the determining step d) is performed during ventilation of the human or the animal. Preferably, the determination of the transdiaphragmatic pressure is based on the variation of the propagation velocity of the shear wave. The linear relation between the transdiaphragmatic pressure and the propagation velocity of the shear wave, generated by the stimulation, may be used to determine the transdiaphragmatic pressure based on the sole value of the propagation velocity of the shear wave.

According to the second variant, the stimulation step a) may further comprise successive emissions of focused ultrasound waves towards the region of interest, each of said successive emissions being performed:
- according to a different axis, and/or
- according to a different focal length,
a determination of spatial organization of muscles fascicles.
Preferably, the determination of spatial organization of muscles fascicles may be based on three-dimensional velocity fields reconstruction.

According to the second variant, during the processing step c), images of diagram tissue previously acquired during the imaging step b), may be processed, by technical means, to measure the mechanical and/or acoustic wave propagating through said diaphragm tissue. A velocity profile of the mechanical and/or acoustic wave is equivalent to a propagation of a movement from one or more parts of the diaphragm to one or more adjacent parts over time.

During the processing step c), the velocity profile of the diaphragm tissue may be processed, by technical means, to measure:
- peaks heights of diaphragm stiffness being indicative of diaphragm contractility, and/or
- a frequency of stimulation being indicative of diaphragm contractility, and/or
- an integral of diaphragm stiffness within the inspiratory time over time being indicative of the work of the diaphragm, and/or
- a diaphragm stiffness-time index computed as the product of the diaphragm stiffness changes per breath within the inspiratory time and the ratio of the inspiratory time over the total respiratory time, being indicative of diaphragm function, and/or
- a slope of the profile being indicative of the contractility of the diaphragm.

According to a second aspect of the invention, there is also provided a device for measuring one or more diaphragmatic functional parameters of a diaphragm of a human or an animal, said device comprising means for processing ultrasound images of one or more moving parts of the diaphragm, said ultrasound images comprising at least 100 images per second of said one or more moving parts of the diaphragm,
said means for processing being arranged and/or configured and/or programmed to measure:
- one or more movements of a given part of the diaphragm over time, and/or
- a propagation of a movement from said one or more parts of the diaphragm to one or more adjacent parts over time, and/or
- a propagation speed of a movement from said one or more parts of the diaphragm to one or more adjacent parts over time, and/or
- one or more movements of different parts of the diaphragm over time, and/or
- an amplitude of a movement of one or different parts of the diaphragm overtime,
- a time separating the stimulation of the diaphragm from the occurrence of a movement of the diaphragm associated to said stimulation;
said means for processing being arranged and/or configured and/or programmed, based on one or more of those previous measurements, to determine one or more functional parameters of the diaphragm.

The one or more moving parts of the diaphragm parts have been set in motion in response to a stimulation of the diaphragm, said stimulation having generating a movement of one or more parts of the diaphragm.

The means for processing may comprise, preferably only, at least one computer, a central and/or a calculation unit and/or a processing unit, an analog electronic circuit (preferably dedicated), a digital electronic circuit (preferably dedicated), and/or a microprocessor (preferably dedicated), and/or software means.

The functional parameters of the diaphragm comprise a contractility and/or a diaphragmatic pressure and/or a diaphragm effort and/or a diaphragm function and/or a diaphragmatic work and/or a nerve conduction velocity and/or a spatial organization of muscle fascicule(s).

The device according to the second aspect of the invention is arranged and/or configured and/or programmed to implement the method, or any step or feature of the method, according to the first aspect of the invention.

According to a third aspect of the invention, there is also provided an apparatus for measuring one or more diaphragmatic functional parameters of a diaphragm of a human or an animal, said apparatus comprising:
- means for stimulating the diaphragm to generate a movement of one or more parts of the diaphragm,
- means for imaging one or more parts of the diaphragm over time, said means for imaging being arranged to:
   - emit at least 100 unfocused ultrasound waves per second towards a region of the human or the animal comprising the one or more parts of the diaphragm to be imaged by the imaging means,
   - detect ultrasound waves reflected and/or scattered by organic tissues of the human or the animal located in the region,
   - process the reflected and/or scattered ultrasound waves over time to generate images,
- means for processing images previously acquired, said means for imaging being arranged and/or configured and/or programmed to measure:
   - one or more movements of a given part of the diaphragm over time, and/or
   - a propagation of a movement from said one or more parts of the diaphragm to one or more adjacent parts over time, and/or
   - a propagation speed of a movement from said one or more parts of the diaphragm to one or more adjacent parts over time, and/or
   - one or more movements of different parts of the diaphragm over time, and/or
   - an amplitude of a movement of one or different parts of the diaphragm overtime,
   - a time separating the stimulation of the diaphragm from the occurrence of a movement of the diaphragm associated to said stimulation;
said means for processing being arranged and/or configured and/or programmed, based on one or more of those previous measurements, to determine one or more functional parameters of the diaphragm.

The means for processing may comprise, preferably only, at least one computer, a central and/or a calculation unit and/or a processing unit, an analog electronic circuit (preferably dedicated), a digital electronic circuit (preferably dedicated), and/or a microprocessor (preferably dedicated), and/or software means.

The means for stimulating may be arranged and/or configured and/or programmed to provide an electrical stimulation and/or a magnetic stimulation.

The means for processing may be arranged and/or configured and/or programmed to determine the amplitude of the movement of the diaphragm based on an intensity of the stimulation.

The means for processing may be arranged and/or configured and/or programmed to determine a nerve conduction velocity.

The means for processing may be arranged and/or configured and/or programmed to determine:
- a contractility of the diaphragm, and/or
- a localized paralysis of the diaphragm.

Preferably, based on a propagation speed of a movement through the diaphragm, the means for processing may be arranged and/or configured and/or programmed to determine:
- a contractility of the diaphragm, and/or
- a localized paralysis of the diaphragm.

The means for stimulating may be arranged and/or configured and/or programmed to provide a mechanical stimulation and/or an acoustic stimulation of one or more parts of the diaphragm to generate a mechanical wave and/or an acoustic wave at said given part, the mechanical wave and/or the acoustic wave propagating towards adjacent parts of said given part, and
- the means for imaging may be arranged and/or configured and/or programmed to image said given part and said adjacent parts through which the mechanical wave and/or the acoustic wave propagate(s).

The means for stimulating may be arranged and/or configured and/or programmed to provide an ultrasonic stimulation, said ultrasonic stimulation comprises an emission of one or more focused ultrasound waves per second towards a given part of the diaphragm, said one or more focused ultrasound waves generate an elastic shear wave at said given part, the elastic shear wave propagates towards adjacent parts of said given part.

The means for imaging may be arranged and/or configured and/or programmed to measure a propagation velocity of the shear wave.

The means for processing may be arranged and/or configured and/or programmed to determine a contractility of the diaphragm based on the propagation velocity of the shear wave.

The apparatus may be arranged and/or configured and/or programmed to measure one or more diaphragmatic functional parameters of a diaphragm of a human or an animal during ventilation of the human or the animal.

The means for processing may be arranged and/or configured and/or programmed to determine a diaphragm work.

The means for processing may be arranged and/or configured and/or programmed to determine a diaphragm activity based on the propagation velocity of the shear waves.

The means for processing may be arranged and/or configured and/or programmed to determine a transdiaphragmatic pressure based on the variation of the propagation velocity of the shear wave.

The means for stimulating may be arranged and/or configured and/or programmed to emit successive focused ultrasound waves towards the region of interest:
- according to a different axis, and/or
- according to a different focal length,
the means for processing may be arranged and/or configured and/or programmed to determine a spatial organization of muscles fascicule(s). Preferably, the determination of spatial organization of muscles fascicles may be based on three-dimensional velocity fields reconstruction.

The functional parameters of the diaphragm comprise a contractility and/or a diaphragmatic pressure and/or a diaphragm function and/or a diaphragm effort and/or a diaphragmatic effort and/or a diaphragmatic work and/or a nerve conduction velocity and/or a spatial organization of muscle fascicule(s).

The apparatus according to the third aspect of the invention is arranged and/or configured and/or programmed to implement the method, or any step or feature of the method, according to the first aspect of the invention.

### Brief description of the drawings

Further objects, features and advantages will appear from the following detailed description of several embodiments of the invention with references to the drawings, in which:
- FIGURE 1 is a schematic representation of the experimental setup used on the human participants,
- FIGURE 2 is a schematic representation of responses of a diaphragm to an electrical or magnetic stimulation,
- FIGURES 3 and 4 are respectively images of diaphragmatic displacements over time in response to an electrical stimulation of high and respectively low intensity,
- FIGURES 5 and 6 are respectively graphs illustrating the amplitude of diaphragmatic displacements according to the intensity of an electrical stimulation and respectively a magnetic stimulation,
- FIGURE 7 shows a set of graphs illustrating a set of measurements performed during static inspiratory efforts against closed airways,
- FIGURE 8 shows a set of graphs illustrating a set of measurements performed during ventilation against inspiratory loading,
- FIGURE 9 shows a set of graphs, each graph is from a different participant, of individual data points illustrating relationship between transdiaphragmatic pressure and diaphragm shear modulus measures from ultrafast ultrasound image in response to an ultrasonic stimulation during submaximal static inspiratory efforts against closed airways,
- FIGURE 10 shows a set of graphs, each graph is from a different participant, of individual data points illustrating relationship individual data points illustrating relationship between transdiaphragmatic pressure and diaphragm shear modulus measures from ultrafast ultrasound image in response to an ultrasonic stimulation during unloaded ventilation and ventilation against inspiratory loading,
- FIGURE 11 shows hysteresis curve between transdiaphragmatic pressure and diaphragm shear modulus measures from ultrafast ultrasound image in response to an ultrasonic stimulation during ventilation against inspiratory loading in one participant.

### Detail description of embodiments of the invention

The embodiments hereinafter described are not restrictive, other embodiments comprising a selection of features described hereinafter may be considered. A selection may comprise features isolated from a set of features (even if this selection is isolated among a sentence comprising other features thereof), if the selection is sufficient to confer a technical advantage or to distinguish the invention form the state of the art. This selection comprises at least a feature, preferably described by its technical function without structural features, or with a part of structural details if this part is sufficient to confer a technical advantage or to distinguish the invention form the state of the art on its own.

Furthermore, the embodiments hereinafter are non-limitative embodiments that are within the scope of the above summary of the invention. Thus, any isolated feature of the below embodiments is considered in combination with the more general or functional steps or features of the above summary of the invention.

### Participants

A total of fifteen healthy participants were studied. All participants gave written informed consent.

### Experimental setup

Participants were studied in a semirecumbent position (40 degrees) with uncast abdomen, breathing through a mouthpiece while wearing a nose clip. The mouthpiece was connected to a three-way valve and pneumotachograph for flow measurement. Mouth pressure (Pmo) was recorded using a differential transducer. Pressure in the lower esophagus (Pes) and pressure in stomach (Pga) were measured using 10-cm balloon catheters, connected separately to differential pressure transducers (model DP45-30; Validyne, Northridge, CA) as previously described. Flow and pressures signals were digitized (Powerlab, ADInstruments, Sydney, Australia) and recorded at a sampling frequency of 2 kHz (Labchart, ADInstruments). Transdiaphragmatic pressure (Pdi) was obtained by online subtraction of Pes from Pga. Ultrasound measurements. Diaphragm ultrasound imaging and shear wave elastography were performed using an Aixplorer Ultrasound scanner (V9.2, Supersonic Imagine, Aix-en-Provence, France) driving a 10-2 MHz linear transducer array (SL10-2, Supersonic Imagine). Settings were defined as follow: B-mode enabled; supersonic shear imaging mode enabled; penetration mode enabled; tissue tuner at 1540 m·s-1; dynamic range at 80 dB; Gain and time gain compensation were tailored for each patient. Scale for Shear Wave Elastography (SWE) was adjusted if required. Sampling rates for B-mode imaging and SWE were 12 and 2 Hz, respectively. A generous amount of water-soluble transmission gel was used during scanning for optimal acoustic coupling and minimal pressure was applied to the transducer in order to limit tissue deformation and modification of ventilator mechanics. The diaphragm was scanned at the right zone of apposition, on the posterior axillary line vertical to the chest wall at the 9th-11th intercostal space. The rotation and angle of the transducer was the finely adjusted to obtain maximal echo intensity from diaphragmatic pleural and peritoneal peritonea. Ultrasound scanned were triggered by the Powerlab for synchronizing ultrasound, flow, and pressures recordings. The transducer position was marked on the skin. Ultrasound measurements were performed by a trained intensivist (MD).

### Study protocol

The study was carried out as follows: i) measurement of maximal static inspiratory pressure (PImax), ii) recordings during apnea at functional residual capacity (FRC), iii) recordings during inspiratory efforts against closed airways, iv) recordings during ventilation against inspiratory loading.

### Maximal static inspiratory pressure

PImax was measured at FRC. At least five trials were performed until three reproducible efforts, with less than 10% variance, were obtained. Maximal Pmo generated amongst the three reproducible trials was defined as PImax.

### Apnea at FRC and static inspiratory efforts against closed airways

During these tasks, the mouthpiece was disconnected from the three-way valve and flow was not monitored. Pressures and Shear Modulus of the diaphragm (SMdi) were measured during about 5s apnea and during inspiratory efforts against closed airways at 10, 20, 30, 40, 50, and 60 % of PImax (two participants did not performed 60% PImax). Both apnea and inspiratory efforts were performed at FRC. Participant (n=13) were asked to reach progressively the target Pmo and to maintain their effort during about 10s. Visual feedback of generated Pmo and guidelines were provided to participants using the built-in software option. Each task was repeated twice. Tasks were alternated with 1-2 min of unloaded breathing.

### Ventilation against inspiratory loading

An in-house developed apparatus was used to perform ventilation against inspiratory elastic loads. Briefly, the device consisted of a cylindrical adjustable pressure chamber connected to a non-rebreathing valve. The negative pressure was generated by a commercially available vacuum cleaner. Pressure in the chamber (Pch) was measured continuously using a differential pressure transducer. The dead space of the device was estimated at about 600 ml. Participants (n=15) underwent a step-wise inspiratory loading protocol at 10, 20, 30, 40 and 50% of PImax (two participants (5, 10) did not performed 50% PImax, one participant (10) did not performed 40% PImax, one participant (7) also performed 60% PImax). Each task was repeated twice. During each task, at least six regular respiratory cycles were recorded. Tasks were alternated with 1-2 min of unloaded breathing.

### Data analysis

Pes, Pga, Pdi, Pmo, Pch and flow were analyzed offline using standardized scripts in MATLAB (Mathworks, Natick, MA, USA). Frames from Bmode and SWE recordings were exported using the ultrasound scanner research pack (Soniclab, v11, Supersonic imagine) and processed using standardized scripts in MATLAB (Mathworks). SMdi was calculated assuming a linear elastic behavior in muscle tissue (4) as SMdi = ρ·Vs2 where ρ is the density of muscle (1000 kg·m-3), and Vs is the shear wave speed or the propagation velocity of the shear wave in m·s-1. The Young Modulus E, and so the tissue elasticity, for soft tissues can be considered as being equal to E = 3SMdi. A rectangular region of interest was manually defined on the first frame of each stack as large as possible between the diaphragm pleural and parietal peritonea. For static inspiratory efforts measurements, signals were manually selected when Pmo was stabilized at the targeted levels. Pressures and SMdi where averaged over the duration of the selected period. Coefficients of variation were computed to assess variability of pressures and SMdi within the selected period. During ventilation against inspiratory loading, maximal SMdi and pressures swing during inspiratory time were computed for each cycle. Cycles were discarded in the case of loss of diaphragm visualization and SMdi > 90 kPa (e.g. when the ROI was in a rib instead of diaphragm). Ultimately, 66 were discarded over 970 recorded cycles. Mean SMdi value during apnea at FRC was subtracted from average and maximal SMdi during static efforts and loaded ventilation, respectively.

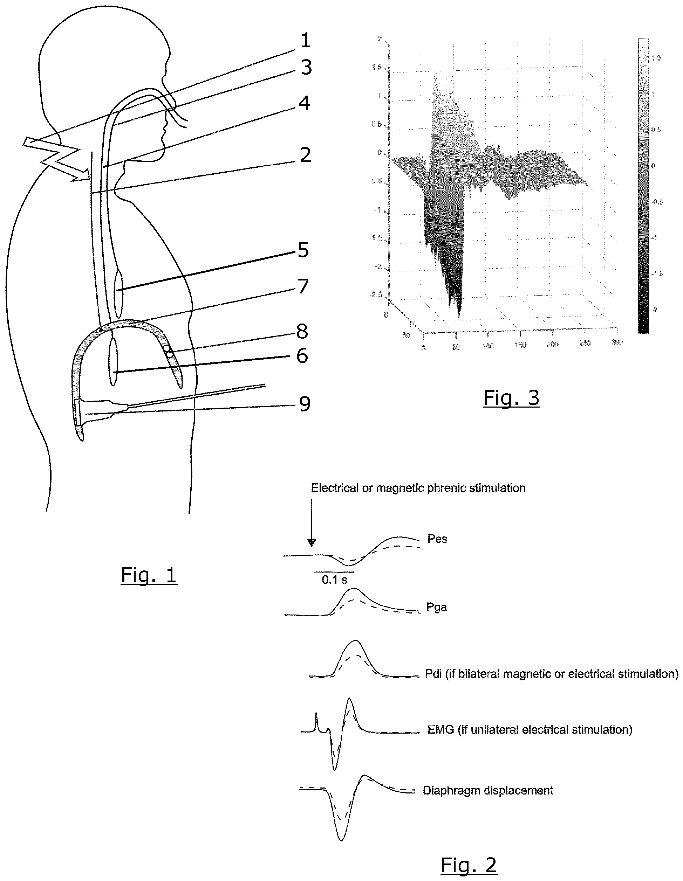

FIGURE 1 shows the schematic representation of the experimental setup used for each participant for a first and a second embodiment. The setup comprises an electrode (not represented) for the electrical or the magnetic stimulation 1 of the phrenic nerve 2. According to the setup the phrenic roots is stimulated in the region of the neck but other regions and/or arrangements may be used.

In order to performed Pdi measurement and correlate Pdi measurement to measurements according to the invention, an esophageal balloon catheter 3 and a gastric balloon catheter 4 are used for Pdi measurements. The balloon in the esophagus 5 located above the diaphragm 7 and the balloon in the stomach 6 under the diaphragm 7 are also illustrated. This invasive protocol is required for Pdi measurements which is the current gold standard for the assessment of diaphragmatic functional parameters. This invasive protocol is not part of the invention but was carried out to prove that the invention provides reliable results and is an alternative to Pdi measurements.

Surface AgCI/Ag electrodes 8 are located on the outer wall of the chest wall about the diaphragm 7 for EMG measurements. The ultrasound probe 9, uses for ultrafast imaging of the diaphragm 7 and stimulating the diaphragm 7, is positioned on the outer wall of the chest wall and moved on any desired location as needed.

The method that comprises emitting at least 100 unfocused ultrasound waves per second, detecting ultrasound waves reflected and/or scattered by organic tissues of the human or the animal located in the region and processing the reflected and/or scattered ultrasound waves over time to generate images is known by ultrafast ultrasound imaging. The apparatus and the setting uses for ultrafast ultrasound imaging are described above.

According to the experiment and as necessary, the processing of ultrasound images is used to measure one or more movements of a given part of the diaphragm over time, and/or a propagation of a movement from said one or more parts of the diaphragm to one or more adjacent parts over time, and/or a propagation speed of a movement from said one or more parts of the diaphragm to one or more adjacent parts over time, namely the SMdi, and/or one or more movements of different parts of the diaphragm over time, and/or an amplitude of a movement of one or different parts of the diaphragm over time, and/or a time separating the stimulation of the diaphragm from the occurrence of a movement of the diaphragm associated to said stimulation. Software, settings and parameters used for measurements, data analysis and determination are described above.

According to the first embodiment illustrating through figures 2 to 6, the stimulation of diaphragm in order to generate a movement of one or more parts of the diaphragm was carried out through electrical or magnetic stimulation 1. This embodiment exhibits great advantages because is the only one that is non-volitional and that can be carried out when the patient is under assisted ventilation and/or under life-support. This embodiment is also the only one that can be carried out when the patient show disorders that prohibit volitional ventilation (or apnea) or simply cannot achieve required respiratory maneuvers.

FIGURE 2 shows a schematic representation of responses of a diaphragm to an electrical or magnetic stimulation 1. The dash line corresponds to the lower stimulation intensity apply and the plain line to the higher stimulation intensity apply. One can see the effects on the Pes, the Pga, the Pdi (if bilateral magnetic or electrical stimulation 1), the EMG (if unilateral electrical stimulation 1) and the diaphragm 7 displacement over time elicited by the electrical or magnetic stimulation 1. One can see the clear differences of the responses for each measured parameters depending on the stimulation intensity.

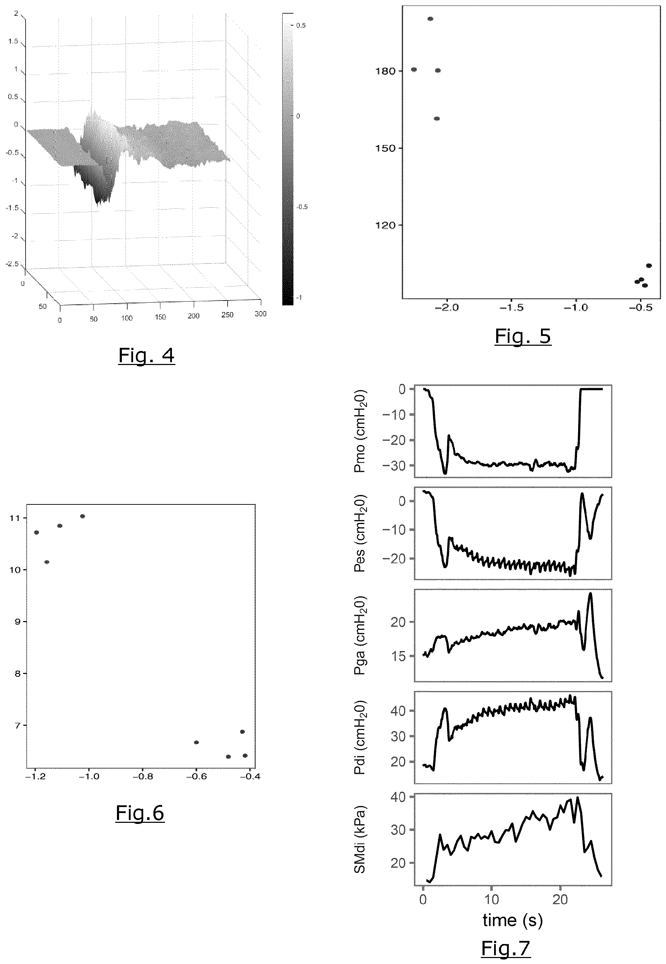

Figures 3 and 4 show diaphragmatic displacements in millimeters (mm), in Y-axis, over time in milliseconds (ms), in X-axis, according to ultrasonic piezo transducers, in Z-axis. Figure 3 shows the image of diaphragmatic displacements amplitude in millimeters over time in response to a supramaximal electrical stimulation. Figure 4 shows the image of diaphragmatic displacements amplitude in micrometers over time in response to an electrical stimulation selected to elicit half the EMG response obtain with supramaximal stimulation. Similar results were obtained through magnetic stimulations 1. These results clearly show that the amplitude of the diaphragm 7 displacements in response to an electrical or magnetic stimulation 1 is linearly correlates to the intensity of the stimulation 1 applied.

A participant has been subjected to supramaximal electrical stimulations 1 and to electrical stimulations selected to elicit half the EMG response obtain with supramaximal stimulation. On figure 5 is shown a graph illustrating amplitudes of diaphragmatic displacements in mm, in X-axis, according to the intensity of the electrical stimulation 1 in millivolts (mV), in Y-axis. The amplitude of diaphragmatic displacements is comprised between 2 and 2.2 mm for a supramaximal electrical stimulation 1 and between 0.45 and 0.55 for electrical stimulation 1 selected to elicit half the EMG response obtain with supramaximal stimulation.

A participant has been subjected to supramaximal magnetic stimulations 1 and to magnetic stimulations selected to elicit half the Pdi response obtain with supramaximal stimulation. Figure 6 shows a graph illustrating amplitudes of diaphragmatic displacements in mm, in X-axis, according to the Pdi. The amplitude is comprised between 1 et 1.2 mm for a supramaximal magnetic stimulation 1 and between 0.45 et 0.55 for magnetic stimulation 1 selected to elicit half the Pdi response obtain with supramaximal stimulation.

The representation of figure 2 also illustrates the determination of the presence of nerve conduction velocity. A delay between the electrical or magnetic stimulation and the displacement of the diaphragm may be measured. This measurement of nerve conduction delay is relative to phrenic nerve conduction velocity.

Thus, among others, the main functional parameter that may be determined from the measurements elicited by such stimulations is the contractility of the diaphragm 7. The contractility of the diaphragm 7 can be assessed based on the intensity of the stimulation 1 from the moment it has established that a linear relation between the intensity stimulation 1 and the amplitude of the movement exists.

According to the second embodiment illustrated through figures 7 to 11, the stimulation of diaphragm in order to generate a movement of one or more parts of the diaphragm was carried out through ultrasonic stimulation. The method that comprises an ultrasonic stimulation comprising an emission of one or more focused ultrasound waves per second towards a given part of an elastic tissue for generating an elastic shear wave that propagates towards adjacent parts of said given part of the elastic tissue in order to observed the shear wave propagation is known as Shear Wave Elastography (SWE). SWE allows measuring the SMdi of a part or the diaphragm or the mean SMdi of the entire diaphragm. The apparatus and the setting used to perform SWE are described above.

FIGURE 7 shows a set of graphs illustrating a set of measurements performed during static inspiratory efforts against closed airways. Pmo, Pes, Pga, Pdi and SMdi were measured during static inspiratory efforts against closed airways. Mean selection duration for averaging data was 8.7 s (SD 3.9). Within selected data, mean of coefficient of variation for Pmo, Pes, Pga, Pdi, and SMdi were 14.2, 9.0, 6.3, 5.4, and 16.2 %, respectively. A clear linear relationship between mean Pdi swing and mean SMdi during all tasks is highlighted and identified. Mean Pdi significantly correlated to mean SMdi in all participants except one (r ranged from 0.60 to 0.92; in participants with significant correlation, all p were < 0.05; R = 0.76, 95% CIs [0.69, 0.82], p < 0.001, r was > 0.70 in ten over thirteen participants).

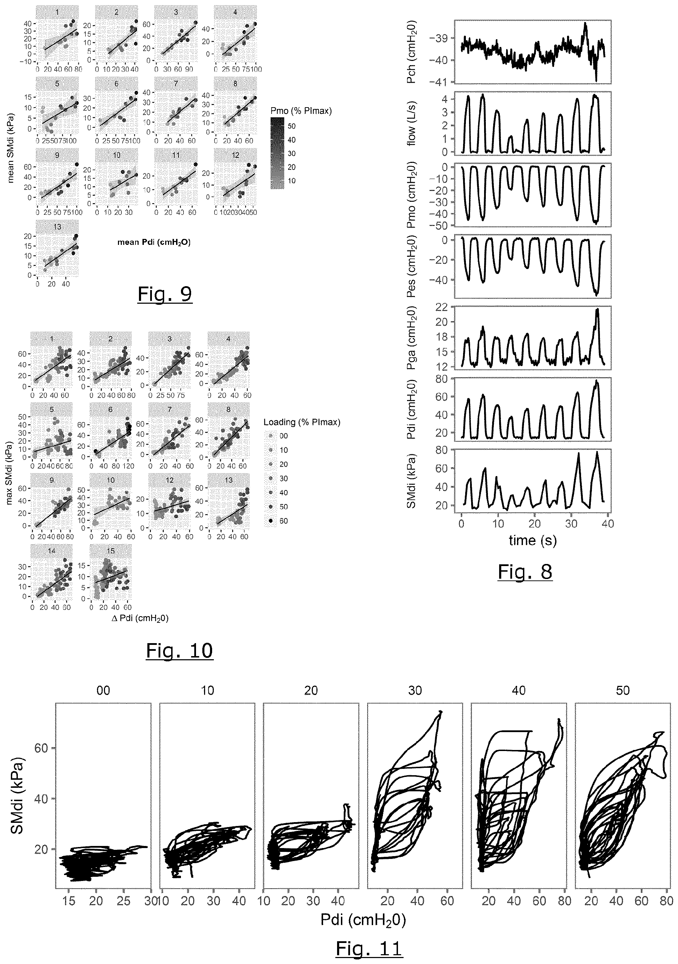

FIGURE 8 shows a set of graphs illustrating a set of measurements performed during ventilation against inspiratory loading. Pch, air flow, Pmo, Pes, Pga, Pdi and SMdi were measured during ventilation against inspiratory loading. Number of cycles analyzed per loading level was 11.8 (SD 3.0). A clear linear relationship between Pdi swing and maximal SMdi for all analysed cycles and all loading tasks is highlighted and identified. Maximal SMdi correlated to Pdi swing in all participants (r ranged from 0.32 to 0.95, all p < 0.01; R = 0.71, 95% CIs [0.68, 0.74], p < 0.001, r was > 0.70 in nine over fifteen participants).

The results illustrate on figure 7 and 8 show that increasing the inspiratory load during both static inspiratory efforts and ventilation against inspiratory loading resulted in an increase in Pdi. These results show strong linear relationship between max SMdi and Pdi swing during ventilation against inspiratory loading. These findings demonstrate for the first time that diaphragm activity can be noninvasively monitored using SWE during breathing.

Thus, among others, the main functional parameter that may be determined is the contractility of the diaphragm 7. The contractility of the diaphragm 7 can be assessed based on propagation velocity of the shear wave from the moment a linear relation between Pdi and SMdi is established.

FIGURES 9 and 10 illustrate, for a set of different participants, the relationship between mean Pdi and mean SMdi for different Pmo, and respectively the relationship between the amplitude of the Pdi according to maximum SMdi for different inspiratory loading. These results demonstrate that diaphragm stiffening is strongly related to the level of diaphragm activation as assessed by standard Pdi measurements. Moreover, high individual correlation coefficients between SMdi and Pdi have been observed in most participants. Slight offset of transducer angle in reference to the direction of muscle fascicles is known to reduce shear modulus value. Therefore, quality criteria for SMdi measurements must be established and adjustment of transducers in the three-dimensional space shall be assisted programmatically to obtain largest SMdi changes during ventilation. Muscle fascicles in reference to the probe might also be estimated using three dimensional SWE measurements.

FIGURE 11 shows hysteresis curves between transdiaphragmatic pressure and diaphragm shear modulus measures from ultrafast ultrasound images in response to an ultrasonic stimulation during ventilation against inspiratory loading in one participant. Figure 11 shows a set of graphs, each graphs corresponds to an inspiratory load, from left to right 0, 10, 20, 30, 40, 50, and 60 % of PImax.

Thus, among others, functional parameters that may be determined are the diaphragm work and the diagram activity.

SMdi coupled with functional respiratory investigations may help to detect diaphragm dysfunction. It might be particularly useful for detecting diaphragm hemi-paralysis. Diaphragm SWE might also particularly relevant within spontaneous breathing trials and/or pressure support ventilation in ventilated patients during the weaning phase. Diaphragm stiffening-time index may also be computed during spontaneous breathing trial.

The invention is not restricted to embodiments described above and numerous adjustments may be achieved within the scope of the invention.

Moreover, features, alternatives and embodiments of the invention may be associated if they are not mutually exclusive of each other.

Thus, in combinable alternatives of previous embodiments:
- the measurement of nerve conduction delay may be performed through any stimulation eliciting a diaphragm contraction, and/or
- according to the first embodiment of the invention, functional parameter that may be determined from the measurements elicited by electrical and/or magnetic stimulations is: the diaphragmatic pressure and/or the diaphragm function and/or the diaphragmatic work, and/or
- according to the first embodiment of the invention, the velocity profile of the diaphragm tissue may be processed, by technical means, to measure:
   - peak heights being indicative of a contractility of the diaphragm, and/or
   - peak areas being indicative of a contractility of the diaphragm, and/or
   - peak widths being indicative of a contractility of the diaphragm, and/or
   - peak slopes being indicative of a contractility of the diaphragm, and/or
   - time to peaks being indicative of a contractility of the diaphragm, and/or
   - an halftime relaxation, and/or
   - an integral of diaphragm displacement within the inspiratory time over time being indicative of a diaphragmatic work, and/or
   - a diaphragm displacement-time index computed as the product of a mean diaphragm displacement per breath within the inspiratory time and the ratio between an inspiratory time and the total respiratory cycle time being indicative of diaphragm function, and/or
- according to the second embodiment, the ultrasonic stimulation may be substitute by any mechanical and/or acoustic stimulation, and/or
- according to the second embodiment, functional parameter that may be determined from the measurements elicited by mechanical and/or acoustic stimulation is: the diaphragmatic pressure and/or the diaphragm function and/or the diaphragmatic work, and/or
- according to the second embodiment, the velocity profile of the diaphragm tissue may be processed, by technical means, to measure:
   - peaks heights of diaphragm stiffness being indicative of diaphragm contractility, and/or
   - a frequency of stimulation being indicative of diaphragm contractility, and/or
   - an integral of diaphragm stiffness within the inspiratory time over time being indicative of the work of the diaphragm, and/or
   - a diaphragm stiffness-time index computed as the product of the diaphragm stiffness changes within the inspiratory time and the ratio of the inspiratory time over the total respiratory time, being indicative of diaphragm function, and/or
   - a slope of the profile being indicative of the contractility of the diaphragm.

## Claims

1. Method for measuring one or more diaphragmatic functional parameters of a diaphragm of a human or an animal, said method comprising the steps consisting of:
- a) a stimulation step by which a stimulation of the diaphragm is provided to generate a movement of one or more parts of the diaphragm,
- b) during the movement of said one or more parts of the diaphragm, imaging one or more parts of the diaphragm over time through an imaging step comprising the steps of:
• emitting at least 100 unfocused ultrasound waves per second towards a region of the human or the animal comprising the one or more parts of the diaphragm to be imaged during the imaging step,
• detecting ultrasound waves reflected and/or scattered by organic tissues of the human or the animal located in the region,
• processing the reflected and/or scattered ultrasound waves over time to generate images by technical means,
- c) processing, by technical means, images previously acquired during the imaging step to measure:
• one or more movements of a given part of the diaphragm over time, and/or
• a propagation of a movement from said one or more parts of the diaphragm to one or more adjacent parts over time, and/or
• a propagation speed of a movement from said one or more parts of the diaphragm to one or more adjacent parts over time, and/or
• one or more movements of different parts of the diaphragm over time, and/or
• an amplitude of a movement of one or different parts of the diaphragm over time, and/or
• a time separating the stimulation of the diaphragm from the occurrence of a movement of the diaphragm associated to said stimulation,
- d) based on one or more measurements of the processing step, determining one or more functional parameters of the diaphragm by technical means.

2. Method according to claim 1, wherein the stimulation of the diaphragm is an electrical and/or a magnetic stimulation.

3. Method according to claim 2, wherein the processing step c) further comprises a measure of the amplitude of the movement of the diaphragm based on an intensity of the stimulation.

4. Method according to claim 2 or 3, wherein the determining step d) further comprises a determination of a nerve conduction velocity.

5. Method according to any of claims 2 to 4, wherein, based on a propagation speed of a movement through the diaphragm, the determining step d) comprises a determination of:
- a contractility of the diaphragm, and/or
- a localized paralysis of the diaphragm.

6. Method according to claim 1, wherein:
- the stimulation step a) further comprises a mechanical stimulation and/or an acoustic stimulation of one or more parts of the diaphragm generating a mechanical wave and/or an acoustic wave at said given part, the mechanical wave and/or the acoustic wave propagating towards adjacent parts of said given part,
- the imaging step b) further comprises imaging said given part and said adjacent parts through which the mechanical wave and/or the acoustic wave propagate(s).

7. Method according to claim 6, wherein the mechanical wave and/or the acoustic stimulation is an ultrasonic stimulation, said ultrasonic stimulation comprising an emission of one or more focused ultrasound waves per second towards a given part of the diaphragm, said one or more focused ultrasound waves generating an elastic shear wave at said given part, the elastic shear wave propagating towards adjacent parts of said given part.

8. Method according to claim 6 or 7, wherein the processing step c) further comprises a measure of a propagation velocity of the shear wave.

9. Method according to any of claims 6 to 8, wherein the determining step d) further comprises a determination of a contractility of the diaphragm based on the propagation velocity of the shear wave.

10. Method according to any of claims 6 to 9, wherein the stimulation step a) is performed during ventilation of the human or the animal.

11. Method according to any of claims 6 to 10, wherein the determining step d) further comprises a determination of a diaphragm work.

12. Method according to any of claims 6 to 11, wherein the determining step d) further comprises a determination of a diaphragm activity based on the propagation velocity of the shear wave.

13. Method according to any of claims 6 to 12, wherein the determining step d) further comprises a determination of a transdiaphragmatic pressure based on the variation of the propagation velocity of the shear wave.

14. Method according to any of claims 6 to 13, wherein the stimulation step a) further comprises successive emissions of focused ultrasound waves towards the region of interest, each of said successive emissions being performed:
• according to a different axis, and/or
• according to a different focal length,
the method further comprises a determination of a spatial organization of muscles fascicles based on three-dimensional velocity fields reconstruction.

15. Method according to any of claims 1 to 14, wherein the functional parameters of the diaphragm comprise a contractility and/or a diaphragmatic pressure and/or a diaphragm function and/or diaphragm effort and/or a diaphragmatic work and/or a nerve conduction velocity and/or an identification of the spatial organization of muscle fascicule(s).

16. Device for measuring one or more diaphragmatic functional parameters of a diaphragm of a human or an animal, said device comprising means for processing ultrasound images of one or more moving parts of the diaphragm, said ultrasound images comprising at least 100 images per second of said one or more moving parts of the diaphragm,
said means for processing being arranged and/or configured and/or programmed to measure:
• one or more movements of a given part of the diaphragm over time, and/or
• a propagation of a movement from said one or more parts of the diaphragm to one or more adjacent parts over time, and/or
• a propagation speed of a movement from said one or more parts of the diaphragm to one or more adjacent parts over time, and/or
• one or more movements of different parts of the diaphragm over time, and/or
• an amplitude of a movement of one or different parts of the diaphragm overtime,
• a time separating the stimulation of the diaphragm from the occurrence of a movement of the diaphragm associated to said stimulation;
said means for processing being arranged and/or configured and/or programmed, based on one or more of those previous measurements, to determine one or more functional parameters of the diaphragm.

17. Apparatus for measuring one or more diaphragmatic functional parameters of a diaphragm of a human or an animal, said apparatus comprising:
- means for stimulating the diaphragm to generate a movement of one or more parts of the diaphragm,
- means for imaging one or more parts of the diaphragm over time, said means for imaging being arranged to:
• emit at least 100 unfocused ultrasound waves per second towards a region of the human or the animal comprising the one or more parts of the diaphragm to be imaged by the imaging means,
• detect ultrasound waves reflected and/or scattered by organic tissues of the human or the animal located in the region,
• process the reflected and/or scattered ultrasound waves over time to generate images,
- means for processing images previously acquired, said means for imaging being arranged and/or configured and/or programmed to measure:
• one or more movements of a given part of the diaphragm over time, and/or
• a propagation of a movement from said one or more parts of the diaphragm to one or more adjacent parts over time, and/or
• a propagation speed of a movement from said one or more parts of the diaphragm to one or more adjacent parts over time, and/or
• one or more movements of different parts of the diaphragm over time, and/or
• an amplitude of a movement of one or different parts of the diaphragm overtime,
• a time separating the stimulation of the diaphragm from the occurrence of a movement of the diaphragm associated to said stimulation;
said means for processing being arranged and/or configured and/or programmed, based on one or more of those previous measurements, to determine one or more functional parameters of the diaphragm.
